Europäisches Patentamt

European Patent Office

Office européen des brevets

(11) Publication number: **0 264 489**
A2

## (12) EUROPEAN PATENT APPLICATION

(21) Application number: 86118167.5

(22) Date of filing: 30.12.86

(51) Int. Cl.³: **C 12 P 21/00**
C 07 K 15/00, C 12 N 15/00
C 12 N 5/00, G 01 N 33/577
//(C12P21/00, C12R1:91)

(30) Priority: 17.10.86 JP 245383/86

(43) Date of publication of application:
27.04.88 Bulletin 88/17

(84) Designated Contracting States:
BE DE FR GB IT

(71) Applicant: Kohjin Co., Ltd.
No. 1-1, Shinbashi 1 chome
Minato-ku Tokyo-to(JP)

(72) Inventor: Kataoka, Katsuyuki
11772-81, Aza-Nooka
Saiki-shi Oita-ken(JP)

(72) Inventor: Yonemura, Masaru
11772-81, Aza Nooka
Saiki-shi Oita-ken(JP)

(72) Inventor: Maruyama, Osamu
11772-81, Aza Nooka
Saiki-shi Oita-ken(JP)

(74) Representative: Türk, Dietmar, Dr. rer. nat. et al,
Türk, Gille + Hrabal Patentanwälte Bruckner Strasse 20
D-4000 Düsseldorf 13(DE)

(54) Monoclonal antibody against human pancreatic amylase, process for preparing the same and process for determining human pancreatic amylase by using the same.

(57) A monoclonal antibody against human pancreatic amylase having a specific reactivity against human pancreatic amylase but completely no or almost no reactivity against human salivary amylase, a process for preparing a monoclonal antibody against human pancreatic amylase, which comprises forming hybridomas by fusion between antibody-producing cells of mice immunized with human pancreatic amylase and myeloma cells of mice, and culturing the hybridomas to collect monoclonal antibody against human pancreatic amylase from culture medium, and a process for immunologically determining human pancreatic amylase by employing the monoclonal antibody with specificity against human pancreatic amylase.

According to the monoclonal antibody and the process of the present invention, only human pancreatic amylase can be selectively determined in a precise and rapid way in the determination of amylase for diagnosis of pancreatic diseases.

FIG. 1

Absorbance at 410 nm vs. Concentration of amylase (fmol/150μl)
—○— P-Amylase
—●— S-Amylase

## MONOCLONAL ANTIBODY AGAINST HUMAN PANCREATIC AMYLASE, PROCESS FOR PREPARING THE SAME AND PROCESS FOR DETERMINING HUMAN PANCREATIC AMYLASE BY USING THE SAME

The present invention relates to a monoclonal antibody against human pancreatic amylase, a process for preparing the same, and a process for immunologically determining human pancreatic amylase.

It has been known that an amount of $\alpha$-amylase present in human blood or urine increases in the case of diseases of pancreas and salivary glands. Therefore, at present, $\alpha$-amylase is an important analysis item in clinical diagnosis.

There are two kinds of isozyme of amylase, i.e. amylase derived from pancreas (hereinafter referred to as "P-amylase") and amylase derived from salivary glands (hereinafter referred to as "S-amylase"). Classification and determination of these isozymes are very important for improving precision of diagnosis. Especially, determination of P-amylase is an important analysis item indispensable in diagnosis of pancreatic diseases and thus the development of a process for determining P-amylase has been desired in the clinical field.

By employing monoclonal antibody against P-amylase of the present invention, P-amylase can be easily determined by means of enzyme immunoassay (EIA) or radio immunoassay (RIA). Therefore, immunological determination of P-amylase in human blood can be easily conducted and the obtained data can be utilized in clinical diagnosis.

For determining amylase isozyme, electrophoretic analysis, a method employing amylase inhibitor, a method by gel filtration and a method by affinity chromatography are known. Among them, only electrophoretic analysis and analysis with amylase inhibitor have been put into a practical use. However,

0264489

both methods have disadvantages such as necessity of special apparatus, complicated and time-wasting procedures in case of the electrophoretic analysis, and insufficient precision in determination due to insufficient specificity of the inhibitor, i.e. amylase inhibitor not only reacts with S-amylase but also cross-reacts with P-amylase at about 20 % in case of the analysis with amylase inhibitor. Therefore, although amylase activity itself is determined in high frequency, the determination of isozyme is not carried out in so much frequency.

Further, although a method for classification and determination of isozyme has been reported by employing monoclonal antibody which inhibits only the enzyme activity of S-amylase but not the activity of P-amylase (hereinafter referred to as "immuno inhibition method", Japanese Unexamined Patent Publication No. 183098/1983), the method has not yet been put into practical use. According to the immuno inhibition method, the activity of S-amylase is inhibited by the antibody specific to S-amylase and the enzyme activity of the remaining P-amylase is measured. However, an affect of impurities and a cross reaction of the antibody with P-amylase lower the precision in determination.

The above-mentioned methods including immuno inhibition method can measure an amount of amylase isozyme only as the enzyme activity but not as an amount of protein. These disadvantages can be overcome by employing the monoclonal antibody against P-amylase of the present invention to carry out enzyme immunoassay or radio immunoassay.

Since the amino acide sequence of P-amylase resembles closely that of S-amylase with elimination of sugar chains, it has hitherto been considered that P-amylase cannot be recognized immunologically. In fact, the antibody against P-amylase has not yet been reported which does not show reactivity against S-amylase.

If the antibody is obtained which does not react with S-amylase at all but specifically reacts with P-amylase, the determination of P-amylase can be easily conducted by employing such antibody to conduct enzyme immunoassay or radio immunoassay. The present inventors studied earnestly based on the above-mentioned consideration in order to overcome the disadvantages of the conventional process for determining amylase isozyme. As a result, the present inventors could prepare the monoclonal antibody which has a high reactivity against P-amylase but almost no reactivity against S-amylase. By conducting enzyme immunoassay with the monoclonal antibody against P-amylase of the present invention, P-amylase can be determined easily with a high precision and as an amount of protein.

According to the present invention, there are provided a monoclonal antibody against human pancreatic amylase having a specific reactivity against human pancreatic amylase but completely no or almost no reactivity against human salivary amylase, a process for preparing the same, which comprises forming hybridomas by fusion between antibody-producing cells of mice immunized with human pancreatic amylase and myeloma cells of mice, and culturing the hybridomas to collect monoclonal antibody against human pancreatic amylase from culture medium, and a process for immunologically determining human pancreatic amylase, which comprises determining human pancreatic amylase by employing the same.

Fig. 1 shows a standard curve in sandwitch assay using the monoclonal antibody 5D4.

Fig. 2 shows a standard curve in sandwitch assay using the monoclonal antibody 8C12.

Fig. 3 shows a standard curve in sandwitch assay using the monoclonal antibody 5D4 as a solid phase and the monoclonal antibody 7F8 as a labelled antibody.

Since reported by G. Köhler and C. Milstein [Nature, 256, 495 to 497 (1975)], there are many reports concerning the preparation of hybridomas. The present inventors tried a number of cell fusions and a screening for obtaining the monoclonal antibody against P-amylase with an excellent property, taking care of the following points in addition to the aforementioned methods:

(1) P-amylase with a high purity is employed as an antigen.

(2) Fetal calf serum employed as a culture medium is lot-checked to carefully select one having a high colony formative efficiency.

(3) Myelomas at a complete logarithmic growing phase are employed.

(4) Growth of hybridomas is fully observed so that a screening and a cloning can be carried out timely.

The lot-check of fetal calf serum in the above point (2) was carried out in consideration of the fact that quality of fetal calf serum, which is an important factor in cell culture, much varies depending on makers or lots, according to a procedure described in "Monoclonal antibody—hybridoma and ELISA, Tatsuo Iwasaki, Kodansha, P 105 - 107" as follows: i.e. RPMI 1640 medium containing 15 % fetal calf serum was prepared for each fetal calf serum. Myeloma cells (X63-Ag8-6.5.3 strain) or self-prepared hybridomas were put on 96-well plates with dillution so that a cell number per well becomes 0.5, 1, 2 or 3, followed by cultivation in $CO_2$ incubator at 37°C. After cultivation for about 14 days, each well was observed with an inverted microscope and a number of well wherein a colony is formed was counted to calculate a colony formation efficiency.

As an example, a procedure for obtaining hybridoma strain is explained in the following.

A solution of 25 to 100 µg of P-amylase highly purified from pancreatic juice dissolved in 0.1 ml of

physiological saline is mixed with complete Freund's adjuvant at 1 : 1 ratio to emulsify. In order to prepare complete emulsion, such procedure is necessary that one of two injectors linked with a connecting needle is charged with an antigen and the other with adjuvant, and both contents are mixed with each other by pushing both pistons alternately. The prepared emulsion is injected intraperitoneally into BALB/c mice of 5 to 10 weeks old. After breeding for 3 to 5 weeks, emulsion of P-amylase and incomplete Freund's adjuvant prepared in the same manner is injected intraperitoneally into the animals. At 3 to 5 weeks interval, the animals are further immunized with booster once or two times in the same way. Two to four weeks after the booster immunization, a solution of 25 to 50 µg of P-amylase in 0.1 mℓ of physiological saline is injected intravenously.

Three days after the final booster, spleen taken out aseptically in clean bench is squashed and suspended in RPMI-1640 medium, which is used for the cell fusion.

On the other hand, mice BALB/c myeloma cell P3-X63-Ag8-U1 strain (hereinafter referred to as "P3U1"), X63-Ag8-6.5.3 strain (hereinafter referred to as "X-653") or P3-NSI-1-Ag4-1 strain is taken out from a freezer. After washing, the myeloma cells are suspended in HT (+ FCS) medium (RPMI-1640 medium containing hypoxanthine, thymidine and 15 % fetal calf serum) at $5 \times 10^5$ cells/mℓ and is cultured in Petri dishes or culture bottles. Culture is continued for 5 days while adding culture medium in accordance with the rate of cell growth every day to give cells at a logarithmic growing phase. Cells are observed for each Petri dish or culture bottle. Among them, cells showing a high growth rate and a good cell condition are selected to use for the cell fusion.

Spleen cells ($1 \times 10^8$ to $2 \times 10^8$) and myeloma cells (about $2 \times 10^7$) are collected by centrifugation and are washed with RPMI-1640 medium three times. After further centrifugation, the supernatant is removed and spleen cells and myeloma cells are thoroughly mixed. After 1 mℓ of 50 % polyethylene glycol 1000 is added to the mixed cells and the mixture is gently stirred for 1 minute, 1 mℓ of RPMI-1640 medium is added to the mixture at 30 seconds interval to add a total amount of 10 mℓ for about 5 minutes. Cells are collected by centrifugation and are suspended in about 60 mℓ of HT (+ FCS) medium. Each 0.1 to 0.15 mℓ of the suspension is put on each well of 96-well plates for the cell culture and the cultivation is conducted in $CO_2$ gas incubator.

Next day, each about 0.1 mℓ of HAT (+ FCS) medium (RPMI-1640 medium containing hypoxanthine, aminopterin, thymidine and 15 % fetal calf serum) is added to each well. After 3 and 6 days, a half amount of the culture medium is removed with suction and thereto each about 0.1 mℓ of fresh HAT (+ FCS) medium is added. After 7 to 14 days, as soon as hybridoma colonies become as large as they can be seen with the naked eye, hybridomas which produce the antibody against P-amylase are screened according to Enzyme Linked Immunosorbent Assay (ELISA) technique employing peroxidase (POD).

In ELISA technique, there are employed commercially available horseradish POD conjugated anti-mouse IgG goat antibody as a labelled antibody, ABTS [2-2'-azino-bis-(3-ethylbenzothyazolin-6-sulfonic acid)] as a coloring reagent, and commercially available S-amylase as it is and P-amylase purified from pancreatic juice as an antigen.

Only hybridoma cells are selected, which produce antibody with a high reactivity against P-amylase but with a low reactivity against S-amylase, and are cloned by conducting a limiting dilution method with thymocytes or spleen cells as a feeder cell more than two times to give monoclonal hybridoma cell lines.

Among the obtained cell lines, those where the growth rate of the cells and the specificity of the antibody are not decreased by subcultures, intraperitoneal culture, frozen storage and the like are selected to give the desired cell lines.

The antibody produced by each cell line can be isolated and purified by the following procedure. After culturing hybridomas in a dish, from $5 \times 10^6$ to $2 \times 10^7$ per mouse of hybridomas are injected intraperitoneally to each mouse to which Pristane (0.5 m$\ell$ per animal) is previously injected intraperitoneally. Mice are breeded and ascites fluid is taken out when the animals have sufficient ascites fluid.

The obtained ascites fluid is subjected to centrifugation to collect the supernatant while the precipitate is discarded. The supernatant is added with sodium azide up to a concentration of 0.1 % and thereto 0.18 g of sodium sulfate per 1 m$\ell$ of ascites fluid is slowly added while stirring the mixture at room temperature. After completion of the addition, the mixture is further stirred for 30 minutes to form sufficient precipitates. The precipitates are collected by centrifugation at 10000 rpm for 10 minutes and are dissolved into 40 mM sodium phosphate buffer containing 30 mM sodium chloride (pH 8.0, hereinafter referred to as "buffer A"). The solution was dialyzed against buffer A at 4°C over a night and the formed precipitate is removed through centrifugation at 10000 rpm for 10 minutes. The supernatant is passed through DEAE-cellulose column previously equilibrated with buffer A and is eluted with the same buffer to give peak I. Further elution with 40 mM sodium phosphate buffer containing 1 M sodium chloride (pH 8.0) gives peak II. The obtained peak I and peak II correspond to IgG fraction and IgM fraction, respectively. After concentrating each fraction, gel filtration is carried out with Sephacryl S-300 column (Pharmacia Inc.) previously equilibrated with 0.1 M sodium phosphate buffer (pH 7.5). IgM is eluted in void volume and IgG is eluted at a fraction corresponding to a molecular weight of $1.5 \times 10^5$. Concentrates of each fraction are employed as a purified antibody.

Each purified antibody was tested for the reaction specificity against S-amylase and P-amylase by the same ELISA technique as in the case of the screening of hybridomas, proving that both purified antibodies showed strong color reaction against P-amylase but completely no or almost no color reaction against S-amylase.

By employing the monoclonal antibody against P-amylase of the present invention, amylase isozyme can be easily determined by EIA or RIA of sandwitch assay technique, competitive assay or the like.

In clinical diagnosis, it is required to determine amylase isozyme at a low concentration with a high precision and without an effect of sera. Therefore, for the labelled antibody used in the sandwitch EIA, anti-sera against amylase is purified by the conventional antibody purification procedure, and Fab is prepared from the resultant according to a method of Ishikawa et al. [Journal of Immunoassay, 4 (3), 209 to 327 (1983)], followed by labelling with POD and further purification using affinity chromatography column wherein S-amylase is immobilized.

By the sandwitch EIA employing the monoclonal antibody against P-amylase of the present invention having a high specificity against P-amylase as a solid phase and POD conjugated Fab purified by affinity chromatography as a labelled antibody, a standard curve with a good linearity is obtained where the monoclonal antibody does not react with S-amylase at all in a wide range of concentration of P-amylase including an average range in the normal human blood.

Detectable concentration is 1 f moℓ per 1 assay tube (1 f moℓ/150 µℓ), which means that the detection can be made up to 1 f moℓ when 10 µℓ of sera is employed as a sample. That is, limit of detectable concentration of P-amylase in sera is 100 f moℓ/mℓ (corresponding to 5.4 ng/mℓ supposing that a molecular weight of P-amylase is 54000). Since P-amylase concentration in normal adult sera is from 24 to 110 ng/mℓ ["Immunological

Determination and Clinical Application of Blood Enzyme", Michio Ogawa, P 59 and P 67 (1984)], the system of the present invention has a sufficient sensitivity for determining P-amylase in sera.

The system of the present invention can specifically determine only P-amylase and induce completely no or almost no cross reaction with S-amylase, which hitherto has been considered to be unavoidable, in a wide range of concentration. Thus, the system of the present invention is not affected by the cross reaction with S-amylase in the determination of P-amylase.

Further, by employing the system of the present invention, so called addition and collection test, where P-amylase is added to sera and recovering percentage of P-amylase is measured, was conducted to give from 89 to 116 % of the recovering percentage. This result shows that P-amylase in sera can be accurately determined by employing the system of the present invention.

Hitherto, anti-sera or monoclonal antibody as a solid phase and anti-sera as a labelled antibody have been usually employed in the sandwitch EIA. However, for obtaining the antibody with a stable quality and the result with an excellent reproducibility, the monoclonal antibody is preferably employed both as a solid phase and as a labelled antibody. Then, the sandwitch assay was also conducted with the system wherein the monoclonal antibody having a high specificity against P-amylase was coated on a solid phase of polystyrene beads, microassay plate or the like and the monoclonal antibody reacting with both P-amylase and S-amylase, i.e. having no specificity against P-amylase, was employed in the state of IgG (i.e., not digested to Fab), in stead of rabbit Fab purified by affinity chromatography, as a labelled antibody, to give a satisfactory standard curve.

Enzyme immunoassay kit or radio immunoassay kit prepared by employing the monoclonal antibody against P-amylase of the present invention can easily separate and determine P-amylase in blood or urine. Amylase includes P-amylase, which increases in blood in pancreatic disease, and S-amylase, which increases in blood in parotiditis and the like. Although the electrophoretic analysis, which requires a complicated procedure, or the analysis with inhibitor, wherein the activity of S-amylase is inhibited and the activity of the remaining P-amylase is measured, have hitherto been commonly employed, both methods have disadvantages as previously mentioned. In this regard, the measurement with the monoclonal antibody against P-amylase of the present invention can provide the following advantages:

(1) Special apparatus is not necessary unlike the electrophoretic analysis and the measurement can be conducted in a quite simple manner.

(2) P-amylase can be determined accurately and precisely since the determination of P-amylase is based on the direct reaction of the monoclonal antibody against P-amylase with P-amylase and not on the measurement of the activity of the remaining P-amylase after inhibiting the activity of S-amylase.

(3) An amount of P-amylase can be measured as an amount of protein and not as the enzyme activity as in the case of the conventional processes. Consequently, the disease can be diagnosed more accurately.

(4) The antibody of the present invention can be utilized for various diagnostic systems. For example, employment in enzyme immunoassay, radio immunoassay or latex agglutination assay can be effective for the determination of blood level of P-amylase.

(5) Since antigenicity is stable as compared with the enzyme activity which is susceptible to inactivation, preservation or handling of samples is quite easy when the measurement is carried out with the method employing the antibody of the present invention.

The present invention is described and explained more particularly by means of the following Examples. However, it should be understood that the present invention is not limited to such Examples and various changes and modifications can be made without departing from the scope of the present invention.

### Example 1

Purified P-amylase as the antigen was prepared according to Matsuura et al. [Journal of Biochemistry, 83, 329 - 332 (1978)] by the following purification procedure.

After adding 5 mg of aprotinin to 200 mℓ of pancreatic juice, the mixture was centrifuged to remove the precipitate. The supernatant was added with 125 mℓ of 20 % corn starch solution, which was prepared by adding 60 g of corn starch to 300 mℓ of 20 % saturated ammonium sulfate solution (pH 8.0) and heating the mixture at 75°C for 30 minutes with occassional stirring, and the mixture was gently stirred at 5°C for 2 hours. Thereafter, the mixture was filtered to collect corn starch to which P-amylase was adsorbed. For extracting P-amylase adsorbed, 120 mℓ of 5 mM calcium acetate buffer containing 5 mM sodium chloride (pH 6.0) was added to the obtained corn starch and the mixture was stirred at room temperature for 1 hour and was filtered to collect the filtrate. This extraction procedure was repeated seven times to give 825 mℓ of the filtrate containing P-amylase. After concentrating the filtrate up to about 100 mℓ by ultrafiltration, the concentrate was dialyzed against 1.5 ℓ of 5 mM calcium acetate buffer containing 5 mM sodium chloride (pH 7.0) at 4°C for 20 hours. After dialysis, centrifugation was conducted to remove insoluble matters to give 108 mℓ of colorless, transparent solution. The obtained solution was passed through DEAE-cellulose column (diameter 2.6 cm, length 31 cm) previously bufferized with 5 mM calcium acetate buffer containing 5 mM sodium chloride (pH 7.0). The pass through fraction was 115 mℓ containing P-amylase. After concentrating the obtained fraction up to about 12 mℓ by ultrafiltration, the resultant was dialyzed against 1 ℓ of 1 mM calcium acetate buffer containing 1 mM sodium chloride (pH 7.0) at 5°C for 20 hours. After dialysis, the obtained solution was passed through Sephadex G-100 column (diameter 2.6 cm, length 71 cm) previously bufferized with 5 mM calcium acetate buffer containing 5 mM sodium chloride (pH 7.0) to give 68 ml of the fraction containing P-amylase. After concentration by ultrafiltration, the resultant was frozen-stored.

The obtained P-amylase (105000 units according to amylase activity assay kit: Amylase-test Wako® , 37 mg of protein) showed one band at the site corresponding to about $6 \times 10^4$ of molecular weight in SDS polyacrylamide gel eletrophoresis.

A solution of 50 µg protein of the obtained purified P-amylase in 0.1 ml of physiological saline was mixed with complete Freund's adjuvant containing mycobacterium (made by Difco Laboratories) at a ratio of 1 : 1 and then the emulsion was injected intraperitoneally to BALB/c mice of 6 weeks age. After 4 weeks, the emulsion was prepared with incomplete Freund's adjuvant (made by Difco Laboratories) in the same manner to carry out the booster injection. After 3 weeks, a solution of 50 µg of P-amylase in 0.1 ml of physiological saline was injected intravenously. Three days after the final booster injection, spleen was taken out asceptically and was squashed and suspended in RPMI-1640 medium, which was used for cell fusion.

On the other hand, mouse BALB/c myeloma cells P3U1 (made by Flow Laboratories Inc. USA) were taken out from frozen storage. After washing, the cells were suspended in HT (+ FCS) medium to give a cell number of $5 \times 10^5$/ml and were cultured on Petri dish. The culture was continued for 5 days with the addition of the culture medium every day to give the cells at logarithmic growing phase. The obtained cells were suspended in RPMI-1640 medium and were used for cell fusion.

Spleen cells ($2 \times 10^8$) and myeloma cells ($2 \times 10^7$) were separately collected by centrifugation and each collected cells were washed with RPMI-1640 medium three times.  After further centrifugation to remove the supernatant, both spleen cells and myeloma cells were sufficiently mixed together.  After 1 mℓ of 50 % polyethylene glycol was added to the mixed cells and the mixture was gently stirred for 1 minute, 1 mℓ of RPMI-1640 medium was added to the mixture at 30 seconds interval to add a total amount of 10 mℓ for 5 minutes. The cells were collected by centrifugation and were suspended in 60 mℓ of HT (+ FCS) medium.  Each 0.12 mℓ of the suspension was seeded each well of 96-well plates (made by Becton Dickinson Labware) for cell culture. Next day, each 0.1 mℓ of HAT (+ FCS) medium was added to each well.  After 3 and 6 days, about a half amount of the culture medium in each well was discarded and thereto each 0.1 mℓ of fresh HAT (+ FCS) medium was added.  After 8 days, wells having a large hybridoma colony were selected and hybridomas secreting the antibody against P-amylase were screened according to ELISA technique employing peroxidase.  A hybridoma clone having a strong reactivity against P-amylase but a weak reactivity against S-amylase was selected and was cloned by conducting a limiting dilution method two times to give hybridoma cell lines 3G1 and 5D4.

The cell lines which are identified in this specification as 3G1 and 5D4 have been deposited ath the Institute for Fermentation, Osaka, Japan (hereinafter referred to  as "IFO") on December 27, 1986 under the accession numbers IFO-50116 and IFO-50117.

Each hybridoma was cultured in a dish and each 1 x $10^7$ cells of hybridoma was injected intraperitoneally to BALB/c mice which were previously injected with Pristance (0.5 mℓ/animal) intraperitoneally.  Mice were breeded for 2 weeks and the accumulated ascites fluid was taken out.

The obtained ascites fluid was subjected to centrifugation to collect the supernatant, to which sodium azide was added up to 0.1 %. To 5 mℓ of the mixture was added slowly 0.9 g of sodium sulfate while stirring at room temperature. After completion of the addition, the mixture was further stirred for 30 minutes to form sufficient precipitates. The precipitates were collected by centrifugation at 10000 rpm for 10 minutes and were dissolved in 5 mℓ of buffer A. The obtained solution was put in a dialysis tube and was dialyzed against buffer A at 4°C for a night. The formed precipitates were removed by centrifugation at 10000 rpm for 10 minutes. The supernatant was passed through DEAE-cellulose column (inner diameter 1.0 cm, length 12 cm) previously bufferized with buffer A and was eluted with the same buffer to give peak I. Further elution with 40 mM sodium phosphate buffer containing 1 M sodium chloride (pH 8.0) gave peak II. The obtained peak I and peak II corresponded to a IgG fraction and a IgM fraction, respectively. After concentrating each fraction with Centricon® (made by Amicon Corporation Scientific), gel filtration was carried out with Sephacryl S-300 column (inner diameter 1.5 cm, length 45 cm) previously bufferized with 0.1 M sodium phosphate buffer (pH 7.5). IgM was eluted in voids volume while IgG was eluted at a fraction corresponding to a molecular weight of $1.5 \times 10^5$.

From the mice to which hybridoma cell line 3G1 was injected, ascites fluid of 8 mℓ per animal was obtained and was purified by the aforementioned purification procedure to give 52 mg protein of IgM. From the mice to which hybridoma cell line 5D4 was injected, ascites fluid of 8 mℓ per animal was obtained and was purified to give 58 mg protein of IgG.

0264489

Each purified antibody was tested for the reaction specificity against S-amylase and P-amylase by the same ELISA technique as in the case of the screening of hybridomas. As a result, the antibody 3G1 showed a strong color reaction against P-amylase but almost no color reaction against S-amylase while the antibody 5D4 showed a strong color reaction against P-amylase but completely no color reaction against S-amylase.

## Example 2

The procedure in Example 1 was repeated except that X-653 (made by Flow Laboratories Inc. USA) was employed as the myeloma cell to give a hybridoma cell line 8C12 and a purified antibody 8C12.

The cell line which is identified in this specification as 8C12 has been deposited at IFO on December 27, 1986 under the accession number IFO-50119.

The following Table 1 and Table 2 show properties of the hybridomas 3G1, 5D4 and 8C12 prepared in Examples 1 and 2, and properties of the antibodies produced by the hybridomas, respectively.

0264489

Table 1

| | Cell line | | |
|---|---|---|---|
| | 3G1 | 5D4 | 8C12 |
| Origin | A hybridoma formed by the fusion between the antibody-forming cell against amylase of mice and the myeloma cell P3U1 | The same as 3G1 | A hybridoma formed by the fusion between the antibody-forming cell against amylase of mice and the myeloma cell X-653 |
| Size | Larger than the myeloma cell and having a diameter of from 15 to 25 μm | The same as 3G1 | The same as 3G1 |
| Function | Producing uniform monoclonal antibody steadily | The same as 3G1 | The same as 3G1 |
| Growth | Showing nearly equal growth rate to the myeloma cell; i.e. the cell number is almost doubled in 18 to 24 hrs Showing almost the same growth rate in abdominal cavity of mice where Pristance in injected | The same as 3G1 | The same as .3G1 |
| Storage | Storage for a long period is possible by suspending the hybridoma in a medium containing 10 % DMSO and storing the tube packed with the suspension in liquid nitrogen; When thawed and cultured after storage, showing the same growth rate and the antibody-forming ability as before the storage | The same as 3G1 | The same as 3G1 |

0264489

| Cell line | | | |
|---|---|---|---|
| | 3G1 | 5D4 | 8C12 |
| Optimum growth condi-tion | Temperature: 37°C, pH 7.0 | The same as 3G1 | The same as 3G1 |

### Table 2

| | | Monoclonal antibody | | |
|---|---|---|---|---|
| | | 3G1 | 5D4 | 8C12 |
| Action | | Generating antigen-antibody reaction against P-amylase | The same as 3G1 | The same as 3G1 |
| Reaction specificity | Reactivity against P-amylase | +++ | +++ | +++ |
| | Reactivity against S-amylase | ± | - | ± |
| Class, Subclass | | IgM | $IgG_1$ | $IgG_1$ |
| Molecular weight | | 900,000 | 150,000 | 150,000 |

In Table 2, the reaction specificity was shown by a degree of the color development in the ELISA technique.

Determination of the class and the subclass was conducted in the following manner.

Each 50 μℓ of a diluted solution of each antibody was put on 5 wells of assay plates (made by Becton Dickinson Labware) coated with P-amylase. After standing at 37°C for 2 hours, each well was washed with phosphate buffer saline (hereinafter referred to as "PBS"). A solution of POD conjugated anti-mouse subclass antibody ($IgG_1$, $IgG_{2a}$, $IgG_{2b}$, $IgG_3$ and IgM, made by Zymed Laboratories Inc.) was added to each well and the reaction was carried out at 37°C for 2 hours. Each well was washed with PBS more than five times. After swishing water off sufficiently, ABTS solution was added to each well for the color development. The subclass can be determined from the well where the color was developed among the wells to which each subclass antibody was added.

The molecular weight was determined by a gel filtration employing Sephacryl S-300.

## Example 3

(1) Preparation of anti-sera against S-amylase

A solution of 0.5 mg of S-amylase (made by Sigma Chemical Co.) dissolved in 0.5 mℓ of physiological saline was mixed with complete Freund's adjuvant containing mycobacterium (made by Difco Laboratories Inc.) at a ratio of 1 : 1 to emulsify and the emulsion was injected subcutaneously in the back of rabbit. One month later, a solution of 0.5 mg of S-amylase dissolved in 0.5 mℓ of physiological saline was injected into ear vein of the rabbit. After 8 days, the animal was sacrificed and whole blood was taken out to give 35 mℓ of sera.

To 20 mℓ of the sera was slowly added 3.6 g of sodium sulfate while stirring at room temperature to form precipitates. The precipitates was collected by

0264489

centrifugation at 10000 rpm for 10 minutes and was dissolved again into 40 mM sodium phosphate buffer containing 30 mM sodium chloride (pH 8.0, hereinafter referred to as "buffer N"). The solution was dialyzed against buffer N at 4°C for a night and the formed precipitates was removed by centrifugation at 10000 rpm for 10 minutes. The supernatant was passed through DEAE-cellulose column previously bufferized with buffer N. The elution with the same buffer gave a IgG fraction. After concentration of the obtained IgG fraction by ultrafiltration, gel filtration was conducted with Sephacryl S-300 column previously bufferized with 0.1 M sodium phosphate buffer (pH 7.5). The desired IgG was eluted at a fraction corresponding to the molecular weight of 150,000. The fraction was concentrated by ultrafiltration.

(2) Preparation of POD conjugated Fab

After rabbit IgG against S-amylase which also equally reacts with the obtained P-amylase was dialyzed against 0.1 M sodium acetate buffer (pH 4.5) for a night, pepsin of a protein amount corresponding to 5 % of that of IgG was added and the incubation was carried out at 37°C for 24 hours. The obtained reaction solution was passed through Ultrogel AcA 44® column (diameter 2.5 cm, length 45 cm, made by LKB Co. Ltd.) previously bufferized with 0.1 M sodium phosphate buffer (pH 7.0) to give a $F(ab)_2$ fraction. After the fraction was concentrated up to 10 mg/ml by ultrafiltration, the concentrate was adjusted to pH 6.0 by adding 0.5 M $NaH_2PO_4$ and was dissolved in 10 mM mercapto-ethylamine. The incubation was conducted at 37°C for 90 minutes to prepare Fab. In order to remove the unreacted mercaptoethylamine, the obtained Fab was passed through Sephadex G-25 column (diameter 1 cm, length 45 cm) previously bufferized with 0.1 M sodium phosphate buffer (pH 6.0) to give a Fab fraction, which was concentrated up to about 200 µl with Centricon® (made by Amicon Corporation Scientific).

On the other hand, maleimide group was introduced into POD. A solution of 0.925 mg of N-(ε-maleimide-caproyloxy)succinimide (made by DOJINDO Laboratories, hereinafter referred to as "EMCS") dissolved in 0.05 mℓ of dimethylformamide was mixed with a solution of 0.95 mℓ of POD dissolved in 0.1 M sodium phosphate buffer (pH 7.0) to conduct incubation at 30°C for 30 minutes. In order to separate POD where maleimide group was introduced from the unreacted EMCS, the reaction solution was passed through Sephadex G-25 column (diameter 1 cm, length 45 cm) previously bufferized with 0.1 M sodium phosphate buffer (pH 6.0) to give a fraction of POD where maleimide group was introduced. The fraction was concentrated up to about 200 µℓ with Centricon® and was used for the reaction with Fab.

Fab and an equimolar amount of POD where maleimide group was introduced were mixed together and the incubation was conducted at 30°C for 1 hour to react the POD with the Fab. For blocking the unreacted maleimide group, 10 mM mercaptoethylamine was added and the incubation was conducted at 30°C for 10 minutes. The reaction solution was passed through Ultrogel AcA 44® column (diameter 1.5 cm, length 45 cm) previously bufferized with 0.1 M sodium phosphate buffer (pH 6.5) to give a POD-Fab conjugated fraction.

(3) Purification of POD conjugated Fab by affinity chromatography

After 10 mg of S-amylase was dialyzed against coupling buffer (0.1 M sodium hydrogencarbonate, pH 8.0) for a night, the centrifugation was conducted at 3000 rpm for 10 minutes to give supernatant containing S-amylase. This S-amylase solution was mixed with CNBr activated Sepharose 4B (made by Pharmacia Co.) and the mixture was stirred at room temperature for 3 hours. By this procedure, not less than 90 % of S-amylase was immobilized on Sepharose 4B. The Sepharose 4B where S-amylase was immobilized was immersed in blocking buffer (Tris-HCℓ buffer, pH 8.0) for a night. The gel was then washed alternately with the coupling buffer and acetate buffer (containing 0.5 M sodium chloride, pH 4.0) four to five times, followed by suspension into 0.01 M sodium phosphate buffer containing 0.1 % bovine serum albumin (pH 7.0).

The column (diameter 3 mm, length 7 mm) was charged with 0.13 mℓ of the above-prepared gel where S-amylase was immobilized, and was washed with 10 mM phosphate buffer (containing 0.1 M sodium chloride and 0.1 % bovine serum albumin, pH 7.0, hereinafter referred to as "buffer B").

Then, the previously prepared solution of POD conjugated Fab (9.08 mg/0.58 mℓ) was passed through the column at a flow rate of 1 mℓ/hr to be adsorbed. After washing the column with 10 mℓ of the buffer B, POD conjugated Fab was eluted by passing 1 mℓ of 3.2 mM hydrochloric acid (pH 2.5) for 10 minutes through the column. The eluate was immediately neutralized with 1 M sodium phosphate buffer (pH 7.0).

The POD conjugated Fab purified by the affinity chromatography was passed through Ultrogel AcA 44® column (diameter 1.5 cm, length 100 cm) to give a fraction of POD conjugated Fab, which was concentrated with Centricon® and was used for sandwitch assay.

(4) Preparation of monoclonal antibody

The hybridoma cell line capable of producing the monoclonal antibody 5D4 against P-amylase prepared in Example 1 was intraperitoneally injected to mice where Pristance was previously injected, and the animals were breeded for 2 weeks so that ascites fluid is accumulated, as in Example 1. The ascites fluid was taken out and was treated with protein A column (made by Bio-Rad Laboratories) to purify the monoclonal antibody.

(5) Sandwitch assay

The monoclonal antibody (0.1 mg/mℓ ) was dissolved in 0.1 M sodium phosphate buffer (pH 7.0) to prepare a coating solution. After polystyrene ball (diameter 3.2 mm, available from Ichico Co. Ltd.) sufficiently washed with distilled water was immersed in the coating solution at 4°C for a night, it was taken out and was immersed in the buffer B for at least one day to conduct blocking.

After washed with 10 mM phosphate buffer (pH 7.0), the polystyrene ball coated with the monoclonal antibody (hereinafter referred to as "antibody-coated ball") was put in a serial dilution of S-amylase and P-amylase prepared with the buffer B to conduct the primary reaction. After washed with 10 mM sodium phosphate buffer (pH 7.0), the antibody-coated ball was then immersed in the affinity-purified POD conjugated Fab solution wherein the POD concentration was adjusted to 50 ng/150 μℓ with the buffer B, to conduct the secondary reaction. After completion of the reaction, the ball was washed with 10 mM sodium phosphate buffer (pH 7.0).

An amount of POD binded to the antibody-coated ball through the antigen-antibody reaction was measured by measuring an optical density at 410 nm in the color reaction employing ABTS as the coloring reagent.

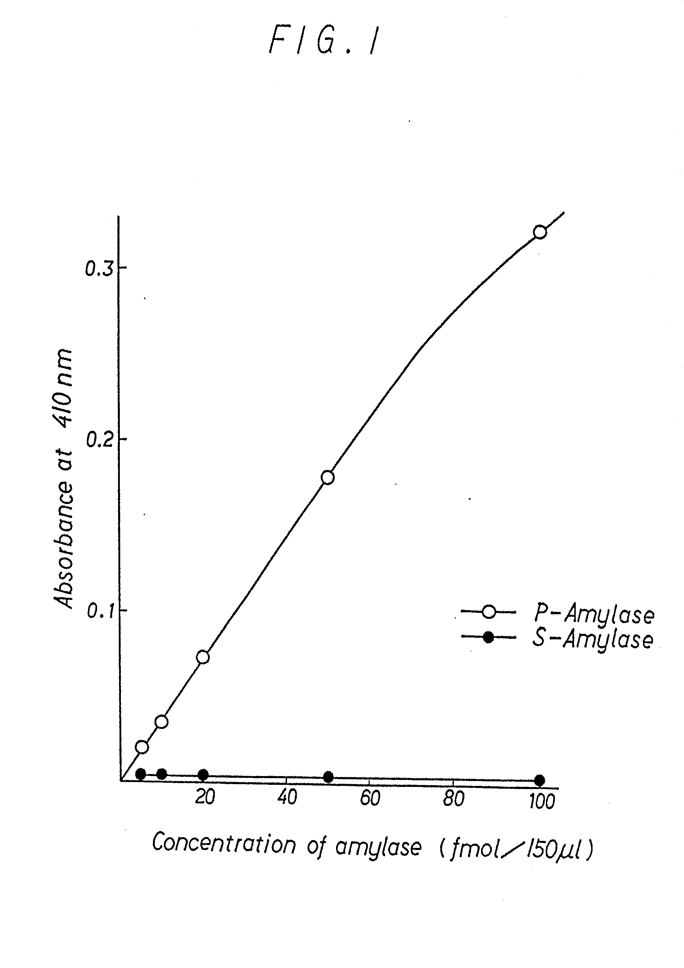

The results are shown in Table 3 and Fig. 1.

Table 3

| An amount of amylase (f moℓ/150 μℓ) | P-Amylase | | S-Amylase | |
|---|---|---|---|---|
| | I* | II** | I* | II** |
| blank | 0.028 | - | 0.028 | - |
| 5 | 0.048 | 0.020 | 0.028 | 0 |
| 10 | 0.063 | 0.035 | 0.028 | 0 |
| 20 | 0.101 | 0.073 | 0.028 | 0 |
| 50 | 0.206 | 0.178 | 0.028 | 0 |
| 100 | 0.350 | 0.322 | 0.028 | 0 |

(note) * Optical density measured at 410 nm in the sandwitch assay employing the monoclonal antibody 5D4

** Value obtained by subtracting the blank value (0.028) from the measured optical density

### Example 4

The sandwitch assay was carried out as in Example 3 except that the antibody 8C12, which was prepared in Example 2 and was frozen-stored, was employed as the monoclonal antibody against P-amylase.

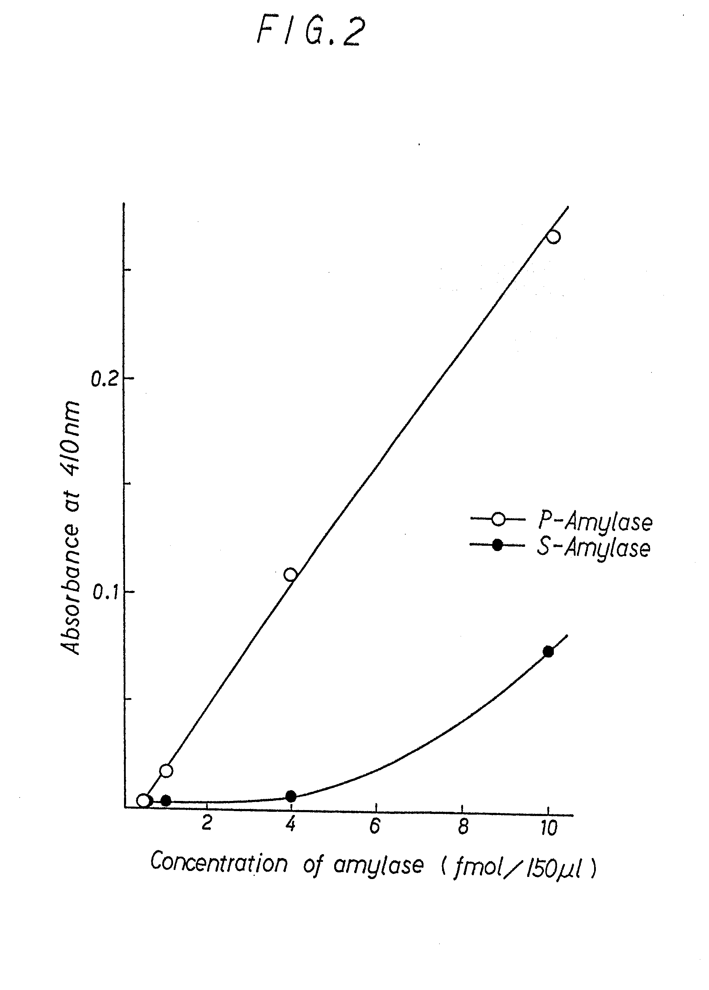

The results are shown in Table 4 and Fig. 2.

### Table 4

| An amount of amylase ($f$ mo$\ell$/150 $\mu\ell$) | P-Amylase | | S-Amylase | |
|---|---|---|---|---|
| | I* | II** | I* | II** |
| blank | 0.029 | - | 0.029 | - |
| 0.4 | 0.032 | 0.003 | 0.029 | 0.000 |
| 1 | 0.046 | 0.017 | 0.030 | 0.001 |
| 4 | 0.138 | 0.109 | 0.035 | 0.006 |
| 10 | 0.297 | 0.268 | 0.105 | 0.076 |

(Note)  *  Optical density measured at 410 nm in the sandwitch assay employing the monoclonal antibody 8C12

** Value obtained by subtracting the blank value (0.029) from the measured optical density

### Example 5

The sandwitch assay was carried out as in Example 3 except that the monoclonal antibody 7F8 (native IgG, instead of Fab), which recognizes both P-amylase and S-amylase almost equally as shown in Table 5, as the labelled antibody and ortho-phenylene diamine, which has a higher sensitivity than ABTS, as a substrate for the coloring reaction were employed.

Table 5

| Properties of the monoclonal antibody 7F8 | | |
|---|---|---|
| Action | | Generating the antigen-antibody reaction against P-amylase |
| Reaction specificity | Reactivity against P-amylase | +++ |
| | Reactivity against S-amylase | +++ |
| Class, Subclass | | IgG$_1$ |
| Molecular weight | | 150,000 |

The monoclonal antibody 7F8 was prepared from hybridoma cell line 7F8 by immunizing mice with P-amylase and screening the hybridomas obtained by cell fusion, as in the case of the other monoclonal antibodies.

The cell line which is identified in this specification as 7F8 has been deposited at IFO on December 27, 1986 under the accession number IFO-50118.

In Table 5, the reaction specificity was shown by a degree of the color development in the ELISA technique.

Determination of the class and the subclass was conducted by the method employing a solution of POD conjugated anti-mouse subclass antibody (IgG$_1$, IgG$_{2a}$, IgG$_{2b}$, IgG$_3$ and IgM, made by Zymed Laboratories Inc.) as the labelled antibody in the ELISA technique.

The molecular weight was determined by a gel filtration employing Sephacryl S-300.

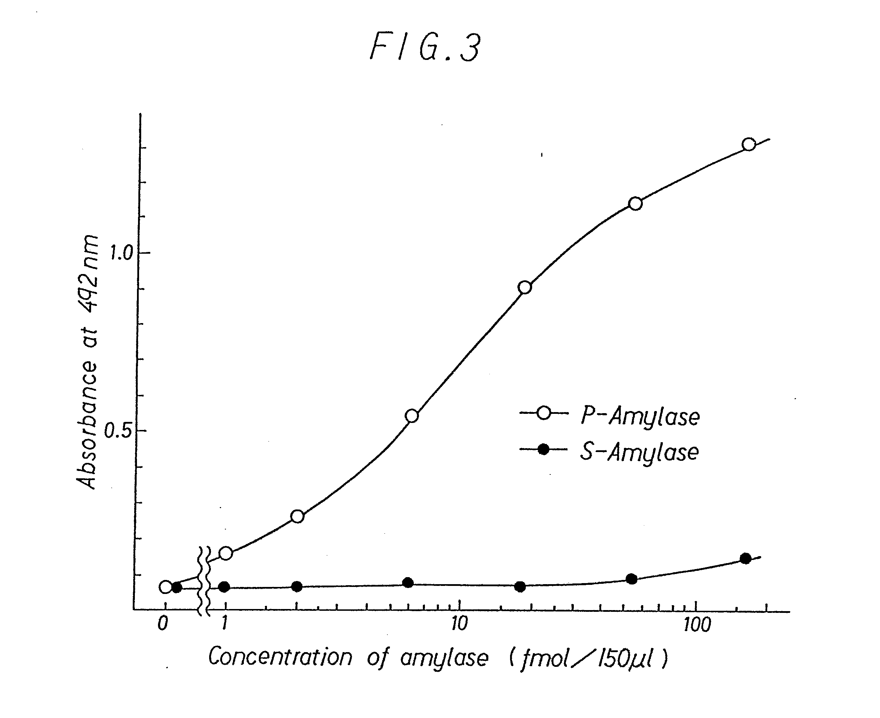

The results of sandwitch assay are shown in Table 6 and Fig. 3.

Table 6 shows measured values of optical density (492 nm) in the sandwitch assay employing the monoclonal antibody 5D4 as the solid phase and the monoclonal antibody 7F8 as the labelled antibody.

Table 6

| An amount of amylase (f moℓ/150 µℓ) | Optical density | |
|---|---|---|
| | P-Amylase | S-Amylase |
| blank (0) | 0.071 | 0.074 |
| 1 | 0.164 | 0.076 |
| 2 | 0.257 | 0.077 |
| 6 | 0.545 | 0.082 |
| 18 | 0.901 | 0.075 |
| 54 | 1.128 | 0.092 |
| 162 | 1.303 | 0.152 |

For demonstrating that the system of the present invention can determine P-amylase in sera, the sandwitch assay was conducted on two kinds of sera A and B containing P-amylase at a different concentration to which P-amylase was further added at a three different concentration, to measure recovering percentage of P-amylase (so called addition and collection test).

The results in Table 7 shows an excellent recovering percentage.

Table 7

| Sera | Contained amount of P-amylase (f moℓ) | Added amount of P-amylase (f moℓ) | Calculated amount (f moℓ) | Measured amount (f moℓ) | Recovering percentage (%) |
|---|---|---|---|---|---|
| A | 13.1 | 6 | 19.1 | 20.5 | 107 |
| | | 18 | 31.1 | 31.0 | 99 |
| | | 54 | 67.1 | 67.0 | 100 |
| B | 36.1 | 6 | 42.1 | 49.0 | 116 |
| | | 18 | 54.1 | 48.0 | 89 |
| | | 54 | 90.1 | 98.0 | 109 |

WHAT WE CLAIM IS:

1. A monoclonal antibody against human pancreatic amylase having a specific reactivity against human pancreatic amylase but completely no or almost no reactivity against human salivary amylase.

2. A process for preparing monoclonal antibody against human pancreatic amylase, which comprises forming hybridomas by fusion between antibody-producing cells of mice immunized with human pancreatic amylase and myeloma cells of mice, and culturing the hybridomas to collect monoclonal antibody against human pancreatic amylase from culture medium.

3. A process for immunologically determining human pancreatic amylase, which comprises determining human pancreatic amylase by employing monoclonal antibody with specificity against human pancreatic amylase.

FIG. 1

0264489

1/3

Concentration of amylase ( fmol／150μl )

## FIG.2

Concentration of amylase ( fmol/150μl )

FIG.3

0264489